# EUROPEAN PATENT APPLICATION

(11) **EP 4 616 877 A1**
(43) Date of publication of application: **17.09.2025**
(21) Application number: 23889144.4
(22) Date of filing: 08.11.2023
(51) Int. Cl.: A61M 1/02, C12M 1/00, C12M 1/12

(54) **KIT FOR CONCENTRATING SOLUTION**

(30) Priority: 08.11.2022 KR 20220147601
(71) Applicant: Rev-Med, Inc., Seongnam-si, Gyeonggi-do 13229 (KR)
(72) Inventor: SHIN, Bong Geun, Suwon-si, Gyeonggi-do 16517 (KR)
(74) Representative: Barrow, Nicholas Martin
(86) International application number: PCT/KR2023/017874
(87) International publication number: WO 2024/101886

(57) **Abstract**

A kit for concentrating solution is provided. A kit for concentrating solution, according to an aspect of the present disclosure, is to generate, from an initial solution including a first material, a second material, and a third material, a concentrated solution in which a concentration of the third material increases, and may comprise: a main housing having a first concentration space therein for separating at least a portion of the first material from the initial solution; a sub housing having a second concentration space therein connected to the first concentration space; and an absorbent member accommodated in the second concentration space so as to selectively absorb the second material.

## Description

### TECHNICAL FIELD

The present disclosure relates to a solution concentration kit, and more specifically, to a solution concentration kit capable of producing a concentrated solution in which a concentration of one material increases from an initial solution.

### BACKGROUND ART

In general, plasma is a neutral, light yellow liquid component obtained by separating blood cells from bone marrow or blood. The plasma contains platelets, growth factors, proteins, carbohydrates, lipids, inorganic salts, and metabolites, in addition to water, and plays a role in maintaining the osmotic pressure and hydrogen ions of cells at a constant level.

Based on this medical evidence, autologous blood therapy and autologous bone marrow therapy are known as autologous platelet regeneration therapies that extract bone marrow or blood from a patient, separate platelets using a centrifugal separator, and then apply the concentrated platelets to wounds or inject the concentrated platelets into ligaments or cartilage.

Here, platelet rich plasma (PRP) obtained by centrifugation of the bone marrow or blood refers to a highly enriched plasma component that is richer in platelets than normal bone marrow or blood, and contains various growth factors that help heal wounds and regenerate and rebuild damaged areas.

In particular, exosomes contained in platelet rich plasma have recently attracted much attention due to their scope and value of use. The exosomes are nanocarriers secreted by all eukaryotic organisms for information exchange between cells, and it has been revealed through prior research that using exosomes derived from the platelet rich plasma for treatment has excellent effects.

In this case, when the platelet rich plasma extracted from a human body is not used immediately in the treatment field, there is a problem that long-term storage is difficult due to the characteristics of blood cells. Therefore, when the platelet rich plasma is activated and then the exosome concentrate is extracted, long-term storage is possible while maintaining the therapeutic effect of the exosome, so the treatment and application range of the exosome can be expanded.

Conventionally, ultra-high-speed centrifugation, chromatography, and precipitation methods have been used to extract exosome concentrates from the platelet rich plasma. However, the above-mentioned methods have the problem of requiring complex equipment and incurring high costs. Therefore, there has been an urgent need for the development of a solution concentration kit that can extract the exosome concentrates easily, at low cost, and with high efficiency.

### DISCLOSURE

### TECHNICAL PROBLEM

The present disclosure has been made in consideration of the above points, and an object of the present disclosure is to provide a solution concentration kit capable of easily, efficiently, and at low cost producing, from an initial solution including first to third materials, a concentrated solution in which the concentration of the third material increases by separating the first and second materials.

Another object of the present disclosure is to provide a solution concentration kit capable of preventing the initial solution and the concentrated solution from coming into contact with the outside air during a process of injecting the initial solution into the kit and a process of extracting the concentrated solution from the outside of the kit.

Another object of the present disclosure is to provide a solution concentration kit capable of sterilizing an internal space by forcibly injecting sterilizing gas into the interior.

Objects of the present disclosure are not limited to the objects mentioned above, and other objects not mentioned will be clearly understood by those skilled in the art to which the present disclosure belongs from the description below.

### TECHNICAL SOLUTION

According to one aspect of the present disclosure, there is provided a solution concentration kit for generating, from an initial solution including a first material, a second material, and a third material, a concentrated solution in which a concentration of the third material increases, the solution concentration kit including a main housing having a first concentration space therein for separating at least a portion of the first material from the initial solution, a sub housing having a second concentration space therein connected to the first concentration space, and an absorbent member accommodated in the second concentration space so as to selectively absorb the second material.

In this case, the absorbent member may further include an isolation member provided between the first concentration space and the second concentration space so that the absorbent member is not movable from the second concentration space to the first concentration space.

In this case, the isolation member may include: a plate-shaped partition portion that partitions the first concentration space and the second concentration space; and a coupling portion formed around the partition portion so as to be coupled to the sub housing.

In this case, a flow hole having a diameter smaller than a diameter of the absorbent member may be formed in the partition portion so that the absorbent member does not pass through.

In this case, the partition portion may have a dome shape convex toward a side of the first concentration space.

In this case, the sub housing may include: a sub body portion having the second concentration space inside; and an outlet portion provided on one side of the sub body portion so that fluid flows out from the second concentration space to an outside.

In this case, the sub body portion may include: a tubular sub body part having one side connected to the first concentration space; and a capturing part provided between the sub body part and the outlet portion so as to capture fluid within the second concentration space, and the capturing part may be provided with an outlet connected to the outlet portion.

In this case, the capturing part may have a shape having a cross-sectional area that decreases toward a side of the outlet portion.

In this case, a slit-shaped discharge groove formed in a flow direction of a fluid and connected to the outlet may be formed in the capturing part.

In this case, a width of the discharge groove may be formed to be smaller than a diameter of the absorbent member.

In this case, the width of the discharge groove may be formed to be smaller than the diameter of the absorbent member expanded by absorbing the second material.

In this case, a plurality of the discharge grooves may be provided, and the plurality of discharge grooves may be disposed radially from a center of the outlet.

In this case, the solution concentration kit may further include: an opening/closing member provided in the first concentration space; and an intermediate housing provided between the main housing and the sub housing so as to move the opening/closing member, in which the first concentration space may be partitioned into a first small space and a second small space disposed closer to the second concentration space than the first small space, and the opening/closing member may be provided so as to open/close a portion where the first small space and the second small space are connected.

In this case, the intermediate housing may include: a first intermediate body portion which is formed of a hollow member inserted into the main housing and includes an outer peripheral portion movably coupled to an inner wall of the main housing; a second intermediate body portion provided on one side of the first intermediate body portion to be coupled to the sub housing; and an opening/closing member support portion provided inside the intermediate housing to support the opening/closing member.

In this case, corresponding screw threads may be formed on the outer peripheral portion of the first intermediate body portion and the inner wall of the main housing, respectively, so as to be screw-coupled.

In this case, an inlet pipe forming a first flow path for fluidly communicating between the first concentration space and the outside may be provided on one side of the main housing, an outlet pipe forming a second flow path for fluidly communicating between the second concentration space and the outside may be provided on one side of the sub housing, a first elastic body having a first slit line formed for opening and closing the first flow path may be coupled to an end portion of the inlet pipe, and a second elastic body having a second slit line formed for opening and closing the second flow path may be coupled to an end portion of the outlet pipe.

In this case, the solution concentration kit may further include: a first sterilizing cap including a first sterilizing cap body that is coupled to the one side of the main housing and a first injection tube that protrudes from the first sterilizing cap body and is penetration-coupled to the first slit line so that the first flow path is opened to the outside; and a second sterilizing cap including a second sterilizing cap body that is coupled to the one side of the sub housing and a second injection tube that protrudes from the second sterilizing cap body and is penetration-coupled to the second slit line so that the second flow path is opened to the outside.

In this case, the solution concentration kit may further include: a first sealing cap including a first sealing cap body that is coupled to the one side of the main housing and a first blocking member that protrudes from the first sealing cap body and blocks the first slit line so that the first flow path is closed; and a second sealing cap including a first sealing cap body that is coupled to the one side of the sub housing and a second blocking member that protrudes from the first sealing cap body and blocks the second slit line so that the second flow path is closed.

In this case, the absorbent member may include a plurality of structures having a spherical shape.

In this case, the second material may be water, and the absorbent member may be made of a super absorbent polymer (SAP) that selectively absorbs water.

In this case, the initial solution may include platelet rich plasma (PRP), and the third material may include an exosome included in the platelet rich plasma.

In this case, the first material may include blood cells remaining in the platelet rich plasma, and the first concentration space may be formed to extend in one direction to separate the first material from the initial solution by the centrifugal separation method.

### ADVANTAGEOUS EFFECTS

The solution concentration kit according to an embodiment of the present disclosure can separate the first material from the main housing and the second material from the sub housing, so that the concentrated solution in which the concentration of the third material increases can be easily produced from the initial solution including the first to third materials.

In addition, since the solution concentration kit according to the embodiment of the present disclosure includes the opening/closing member that can isolate the first isolation space by partitioning the first concentration space into the first small space and the second small space, the initial solution in the first isolation space of the sub housing is separated into a separation solution including the first material and an intermediate solution in which the first material is separated, and is positioned in the first small space and the second small space, respectively, and the opening/closing member is used to block the portion where the first small space and the second small space are connected, thereby easily producing the intermediate solution in which the first material is separated from the initial solution while isolating the intermediate solution from the first material.

In addition, since the solution concentration kit according to the embodiment of the present disclosure includes the absorbent member accommodated in the second concentration space of the sub housing, the concentrated solution in which the concentration of the third material increases can be easily and conveniently produced without separate equipment from the intermediate solution by selectively separating the second material from the intermediate solution.

In addition, the solution concentration kit according to the embodiment of the present disclosure has the isolation member provided between the first concentration space of the main housing and the second concentration space of the sub housing, so that the absorbent member can be prevented from moving from the second concentration space to the first concentration space, thereby preventing the absorbent member from reacting first with a solution other than the intermediate solution.

In addition, in the solution concentration kit according to the embodiment of the present disclosure, since the absorbent member is made of a super absorbent polymer (SAP) that can selectively absorb water, water can be selectively separated from platelet rich plasma (PRP), thereby easily and conveniently producing an exosome concentrate at low cost and with high efficiency.

In addition, since the solution concentration kit according to the embodiment of the present disclosure is provided with the discharge groove connected to the outlet in the capturing part of the sub housing, even when the outlet is covered by the absorbent member, the generated concentrated solution can be easily extracted to the outside.

In addition, in the solution concentration kit according to the embodiment of the present disclosure, since the first and second elastic bodies having the slit lines are coupled to one end of the inlet pipe of the main housing and one end of the outlet pipe of the sub housing, respectively, the initial solution and the concentrated solution can be prevented from coming into contact with the outside air during the process in which the initial solution is injected into the first concentration space or the concentrated solution is extracted outside the second concentration space.

In addition, since the concentration kit according to the embodiment of the present disclosure includes a sterilizing cap having an injection tube that can be fit-coupled into a slit portion of the elastic body so that the flow path through which a fluid can flow is formed inside, the first concentration space and the second concentration space can be sterilized by forcibly injecting sterilizing gas into the inside.

Effects of the present disclosure are not limited to the effects described above, and effects not mentioned can be clearly understood by a person skilled in the art from this specification and the attached drawings.

### DESCRIPTION OF DRAWINGS

FIG. 1 is a perspective view of a solution concentration kit according to an embodiment of the present disclosure as viewed from below.
FIG. 2 is a perspective view of the solution concentration kit according to the embodiment of the present disclosure as viewed from above.
FIG. 3 is a side view of the solution concentration kit according to the embodiment of the present disclosure.
FIG. 4 is an exploded perspective view of the solution concentration kit according to the embodiment of the present disclosure as viewed from above.
FIG. 5 is an exploded perspective view of the solution concentration kit according to the embodiment of the present disclosure as viewed from below.
FIG. 6 is a vertical cross-sectional view of a solution concentration kit according to the embodiment of the present disclosure.
FIG. 7 is an exploded perspective view of an opening/closing member and an intermediate housing according to the embodiment of the present disclosure, and in this case, the intermediate housing is illustrated in cross-section so that the inside can be seen.
FIG. 8 is an enlarged view of a part of FIG. 6 to explain the operation of the opening/closing member according to the embodiment of the present disclosure.
FIG. 9 is a perspective view illustrating the inside of a sub housing according to the embodiment of the present disclosure in cross section.
FIG. 10 is an exploded perspective view of a connection unit, a sterilizing cap, and a sealing cap according to the embodiment of the present disclosure as viewed from below.
FIG. 11 is an exploded perspective view of the connection unit, the sterilizing cap, and the sealing cap according to the embodiment of the present disclosure as viewed from above.
FIG. 12 is a vertical cross-sectional view illustrating a part of the solution concentration kit in which the connection unit and the sterilizing cap are coupled according to the embodiment of the present disclosure.
FIG. 13 is a vertical cross-sectional view illustrating a part of the solution concentration kit in which the connection unit and the sealing cap are coupled according to the embodiment of the present disclosure.
FIG. 14 is a schematic view illustrating a state in which a packaging case including the solution concentration kit according to the embodiment of the present disclosure is stored and sterilized in a sterilization chamber.
FIG. 15 is a view for explaining a process in which the interior of the solution concentration kit according to the embodiment of the present disclosure is sterilized by sterilizing gas introduced through a first sealing cap and a second sealing cap.
FIG. 16 is a view illustrating a state in which an initial solution is injected into a first concentration space of the solution concentration kit according to the embodiment of the present disclosure.
FIG. 17 is a view for explaining a process in which an opening/closing member of the solution concentration kit according to the embodiment of the present disclosure isolates a first small space and a second small space of the first concentration space.
FIG. 18 is a view illustrating a process in which a second connection unit of the solution concentration kit according to the embodiment of the present disclosure is separated from a second sterilizing cap and then coupled with the second sealing cap.
FIG. 19 and FIG. 20 are views for explaining a process in which an intermediate solution is concentrated in a second concentration space of the solution concentration kit according to the embodiment of the present disclosure to generate a concentrated solution.
FIG. 21 is a view for explaining a process in which the concentrated solution generated in the second concentration space of the solution concentration kit according to the embodiment of the present disclosure is taken out to the outside.

### MODES OF THE INVENTION

Hereinafter, with reference to the attached drawings, embodiments of the present disclosure will be described in detail so that those skilled in the art can easily practice the present disclosure. The present disclosure may be implemented in various different forms and is not limited to the embodiments described herein. In order to clearly describe the present disclosure, parts that are not related to the description are omitted in the drawings, and the same reference numerals are assigned to the same or similar components throughout the specification.

The words and terms used in this specification and claims should not be construed as limited to their usual or dictionary meanings, but should be interpreted as having meanings and concepts consistent with the technical idea of the present disclosure, in accordance with the principles by which the inventor can define terms and concepts in order to best describe his or her disclosure.

In this specification, it should be understood that terms such as "include" or "have" are intended to describe the presence of a feature, number, step, operation, component, part or combination thereof described in the specification, but do not exclude in advance the possibility of the presence or addition of one or more other features, numbers, steps, operations, components, parts or combinations thereof.

When a component is said to be "in front of", "behind", "above", or "below" another component, unless there are special circumstances, it includes not only being positioned "in front of", "behind", "above", or "below" the other component in direct contact with it, but also cases where there are other components intervening therebetween. Furthermore, when a component is said to be "connected" to another component, unless there are special circumstances, it includes cases where they are indirectly connected to each other as well as cases where they are directly connected to each other.

A solution concentration kit according to an embodiment of the present disclosure is a kit for generating a concentrated solution in which the concentration of the remaining materials increases by separating at least two materials from an initial solution in which a plurality of materials is mixed.

For example, the initial solution may be a solution including a first material, a second material, and a third material, and the solution concentration kit according to the present embodiment may be configured to generate a concentrated solution in which a concentration of the third material increases by separating at least a portion of the first material and at least a portion of the second material from the initial solution.

In this case, as an example, the initial solution may be platelet rich plasma (PRP), the first material may be blood cells including red blood cells, white blood cells, or the like contained in the plasma, the second material may be water, and the third material may be an exosome contained in the plasma. However, a solution concentration kit 1 according to an embodiment of the present disclosure is not limited to producing an exosome concentrate from platelet rich plasma.

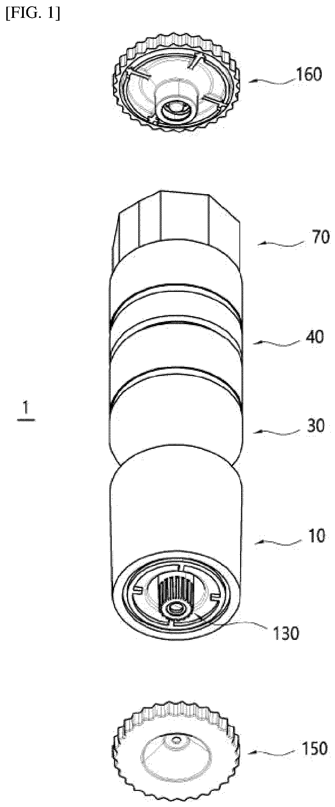

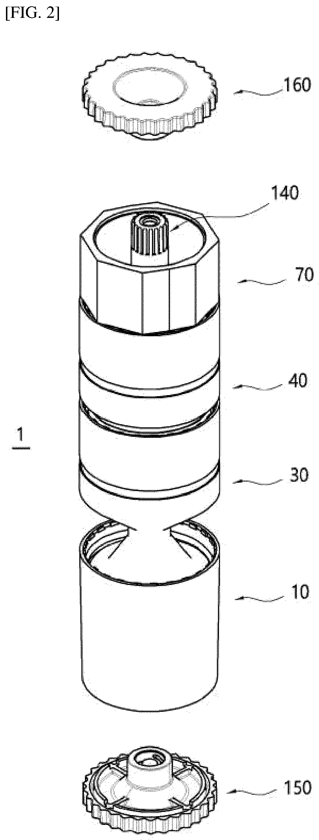

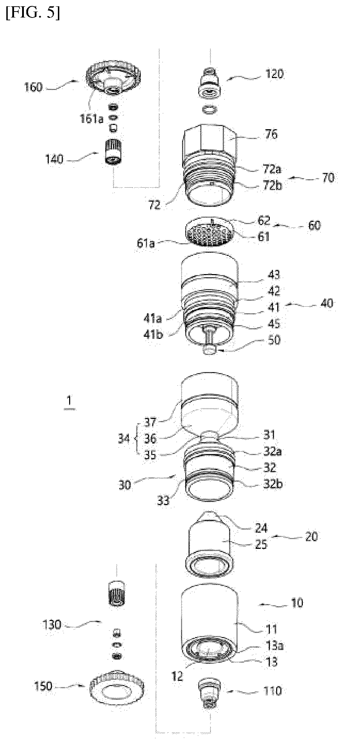

Referring to FIGS. 1 to 5, the solution concentration kit 1 according to the embodiment of the present disclosure may include a cover member 10, an inlet member 20, a main housing 30, an intermediate housing 40, an opening/closing member 50, an isolation member 60, and a sub housing 70 that may be sequentially coupled in one direction, for example, in an up-down direction with respect to FIG. 1.

In the present embodiment, the initial solution may be injected from the outside into the main housing 30 through the inlet member 20. As will be described in detail later, the main housing 30 is configured to separate at least a portion of the first material from the initial solution. In this case, the first material may be separated from the initial solution by a predetermined method, for example, a centrifugal separation method. Hereinafter, a solution in which at least a portion of the first material is separated inside the main housing 30 is referred to as an intermediate solution.

The intermediate solution may flow out of the main housing 30 and into the sub housing 70. As will be described in detail later, the sub housing 70 is configured to separate at least a portion of the second material included in the intermediate solution. An absorbent member capable of selectively absorbing the second material may be provided inside the sub housing 70, and may be configured to separate at least a portion of the second material from the intermediate solution.

A concentrated solution in which at least a portion of the second material is separated and the concentration of the third material increases may be extracted from one side of the sub housing 70, for example, the upper side with respect to FIG. 1.

In this way, the solution concentration kit 1 according to the embodiment of the present disclosure may discharge the concentrated solution to the other side simply by operating in a predetermined manner after the initial solution is injected into one side, so that the concentrated solution in which the concentration of the third material increases may be produced simply and easily without special knowledge or professional tools.

Meanwhile, the solution concentration kit 1 according to the embodiment of the present disclosure may further include a first coupling unit 110, a second coupling unit 120, a first sterilizing cap 130, a second sterilizing cap 140, a first sealing cap 150, and a second sealing cap 160.

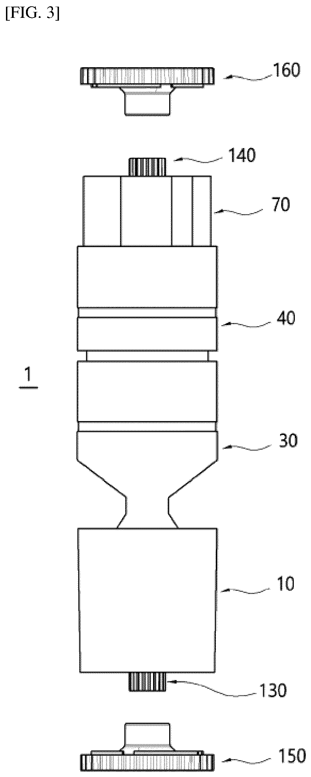

The first coupling unit 110 is coupled to one side of the inlet member 20, for example, the lower side with reference to FIG. 3, and is configured to open and close a first flow path formed inside the inlet member 20 to connect the inside and the outside of the main housing 30. The first coupling unit 110 coupled to the inlet member 20 may be selectively coupled to either the first sterilizing cap 130 or the first sealing cap 150.

The first sterilizing cap 130 coupled with the first coupling unit 110 may open the first flow path so that an external sterilizing gas may be forcibly injected into the interior of the solution concentration kit 1. The first sealing cap 150 coupled with the first coupling unit 110 may close the first flow path so that the interior of the solution concentration kit 1 may be isolated from the exterior.

The second coupling unit 120 is configured to open and close a second flow path formed to connect the inside and the outside of the sub housing 70 by coupling to one side of the sub housing 70, for example, the upper side with reference to FIG. 3. The second coupling unit 120 coupled with the sub housing 70 may be selectively coupled to either the second sterilizing cap 140 or the second sealing cap 160.

The second sterilizing cap 140 coupled with the second coupling unit 120 may open the second flow path so that external sterilizing gas may be forcibly injected into the interior of the solution concentration kit 1. The second sealing cap 160 coupled with the second coupling unit 120 may close the second flow path so that the interior of the solution concentration kit 1 may be isolated from the exterior.

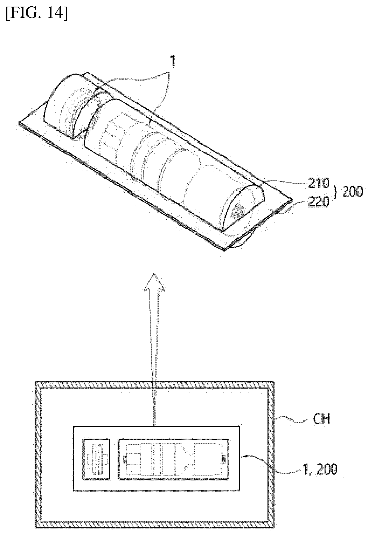

The specific functions of the first coupling unit 110, the second coupling unit 120, the first sterilizing cap 130, the second sterilizing cap 140, the first sealing cap 150, and the second sealing cap 160 will be described later with reference to FIGS. 14 to 21.

Hereinafter, each component of the solution concentration kit according to the embodiment of the present disclosure will be specifically described.

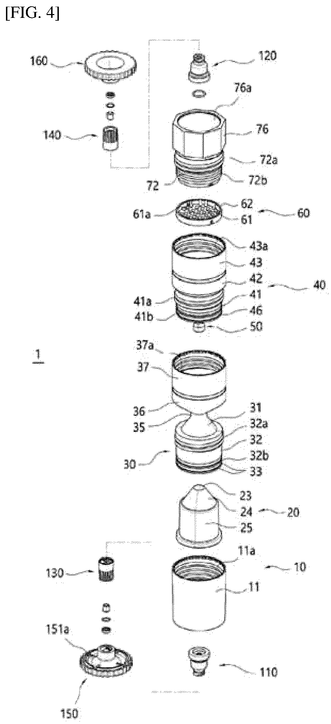

Referring to FIGS. 4 to 6, the solution concentration kit 1 according to the embodiment of the present disclosure includes the cover member 10. The cover member 10 is configured to cover and protect one side of the solution concentration kit 1, for example, the lower side with reference to FIG. 3, and may be made of a material such as plastic having a predetermined rigidity.

The cover member 10 may include an outer cover portion 11, an inner cover portion 12, an outer bottom portion 13, and an inner bottom portion 14. The outer cover portion 11 may be formed of a tubular member. In the illustrated embodiment, the outer cover portion 11 has a cylindrical shape extending in the up-down direction.

The inner peripheral portion of the outer cover portion 11 may be provided with a cover member-side screw thread 11a to which a first main housing-side screw thread 32a of the main housing 30 described later may be correspondingly coupled. In the illustrated embodiment, the cover member-side screw thread 11a is formed on the upper side of the inner peripheral portion of the outer cover portion 11. Accordingly, the cover member 10 may be coupled while covering one side of the main housing 30.

The inner cover portion 12 is provided inside the outer cover portion 11. The inner cover portion 12 may be formed of a cylindrical member having a diameter slightly smaller than that of the outer cover portion 11 and arranged parallel to the outer cover portion 11. In this case, the inner cover portion 12 may have a shorter length than the outer cover portion 11 so as not to protrude outside the outer cover portion 11. An inlet member 20 described later and one side of the main housing 30 may be inserted and connected between the outer cover portion 11 and the inner cover portion 12.

Between the outer cover portion 11 and the inner cover portion 12, the outer bottom portion 13 may be provided to cover and protect the end portions of the inlet member 20 and the main housing 30. To this end, the outer bottom portion 13 may be formed as a donut-shaped disc connecting one end portion of the outer cover portion 11 and one end portion of the inner cover portion 12, for example, the lower end portions with reference to FIG. 3. In this case, an engaging protrusion insertion groove 13a may be formed on the outer surface of the outer bottom portion 13 along the circumferential direction, into which a first sealing cap-side engaging protrusion 151a of the first sealing cap 150 described later may be inserted and coupled.

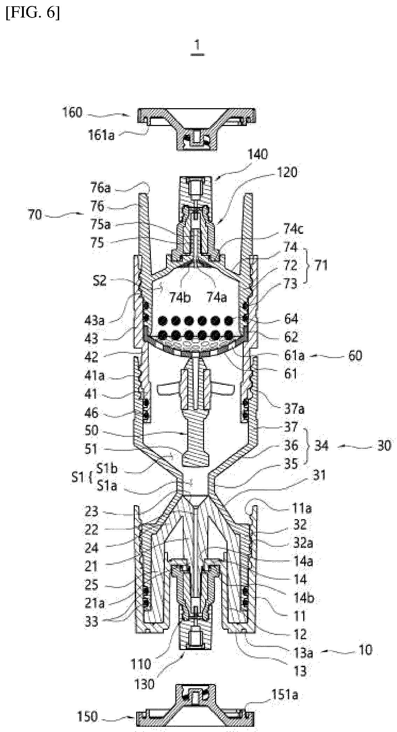

Referring to FIG. 6, an inner bottom portion 14 may be provided inside the inner cover portion 12. The inner bottom portion 14 may be formed of a disc-shaped member provided to block the hollow space of the inner cover portion 12.

The inner bottom portion 14 is configured to provide a base for coupling the first coupling unit 110 described below and an inlet portion 21 of the inlet member 20. To this end, the inner bottom portion 14 may be provided with an inlet member through hole 14a through which the inlet portion 21 of the inlet member 20 may be penetration-coupled. One surface of the inner bottom portion 14, for example, a lower surface thereof with reference to FIG. 6, may be provided with a holder insertion groove 14b to which the first coupling unit 110 may be fit-coupled. The holder insertion groove 14b may be formed along the periphery of the inlet member through hole 14a.

Meanwhile, referring again to FIGS. 4 to 6, in the present embodiment, the inlet member 20 may be coupled to the cover member 10. The inlet member 20 is configured to form the first flow path that may connect the inside of the main housing 30 described below to the outside. Accordingly, the initial solution may be injected from the outside into the inside of the main housing 30 through the inlet member 20.

The inlet member 20 may include the inlet portion 21, an insertion fixing portion 24, and an engagement fixing portion 25. The inlet portion 21 may be configured to form a part of the first flow path and may be composed of a member that has a tubular shape that extends in one direction, for example, in the up-down direction with reference to FIG. 6. In other words, the inlet portion 21 may be provided with an inflow hole 22 that extends in the up-down direction.

As described above, the inlet portion 21 may be penetration-coupled to the inlet member through hole 14a of the inner bottom portion 14 so that one end portion protrudes into the inner cover portion 12 of the cover member 10. In this case, an inlet portion-side engagement portion 21a formed in a step manner so that the engagement portion may be engaged and fixed to the inner bottom portion 14 may be provided on the outer peripheral portion of the inlet portion 21.

The other side of the inlet portion 21 may be provided with an enlarged hole 23 connected to the inflow hole 22. The enlarged hole 23 may be formed to have a cross-sectional area that becomes wider as it goes toward the other end portion of the inlet portion 21. In the illustrated embodiment, the enlarged hole 23 has a funnel shape facing upward. Accordingly, the liquid flowing in through the inflow hole 22 of the inlet portion 21 may more easily flow into the interior of the main housing 30.

Referring to FIGS. 4 to 6, an insertion fixing portion 24 may be formed to be inclined in an outward direction on the outer side of the inlet portion 21. The insertion fixing portion 24 is configured to be inserted and fixed into an inlet member insertion portion 31 of the main housing 30 described later, and may be formed to correspond to the shape of the inlet member insertion portion 31. In the illustrated embodiment, the inlet member insertion portion 31 is formed in a funnel shape inclined in a downward direction.

The engagement fixing portion 25 is provided on one side of the insertion fixing portion 24. The engagement fixing portion 25 may be formed as a cylindrical member extending in one direction, that is, in the up-down direction with reference to FIG. 6, so as to be inserted between the outer cover portion 11 and the inner cover portion 12 of the cover member 10. The engagement fixing portion 25 may have an end portion that is bent. Accordingly, the end portion of the engagement fixing portion 25 may be engaged with one end portion of the main housing 30 coupled to the inside of the cover member 10, thereby being fixed between the outer cover portion 11 and the inner cover portion 12.

Meanwhile, in the present embodiment, the inlet member 20 is configured to be provided separately from the cover member 10 described above and the main housing 30 described below, but, if necessary, the inlet member 20 may be provided as a single member formed integrally with either the cover member 10 or the main housing 30. Of course, the cover member 10, the inlet member 20, and the main housing 30 may also be provided as a single member.

The main housing 30 is provided on one side of the inlet member 20. As described above, the main housing 30 is configured to separate at least a portion of the first material from the initial solution, and may have an hourglass shape that is positioned in one direction, that is, in the up-down direction with reference to FIG. 4, when viewed as a whole.

The lower portion of the main housing 30 may be formed of an inlet member insertion portion 31 and a cover member coupling portion 32 that are coupled with the above-described cover member 10 and inlet member 20. As described above, the inlet member insertion portion 31 is a portion where the inlet member 20 is inserted and fixed, and may have a funnel shape that opens toward the inlet member 20.

The cover member coupling portion 32 extending between the outer cover portion 11 and the inner cover portion 12 of the cover member 10 may be provided on one side of the inlet member insertion portion 31, for example, on the lower side with reference to FIG. 4. The cover member coupling portion 32 is configured to couple the cover member 10 and the main housing 30.

To this end, the outer peripheral portion of the cover member coupling portion 32 is in contact with the inner peripheral portion of the outer cover portion 11 of the cover member 10, and the first main housing-side screw thread 32a that is coupled correspondingly to the cover member-side screw thread 11a of the above-described outer cover portion 11 may be formed on the outer peripheral portion of the cover member coupling portion 32.

In this case, in order to prevent fluid from leaking between the cover member coupling portion 32 and the outer cover portion 11, a first sealing member groove 32b is provided on the outer peripheral portion of the cover member coupling portion 32, and a first sealing member 33 may be installed in the first sealing member groove 32b. The first sealing member 33 may be formed of, for example, an O-ring. The first sealing member groove 32b and the first sealing member 33 may be provided in multiple along the extension direction of the cover member coupling portion 32. Of course, it may also be possible to configure the groove in which the first sealing member 33 is installed to be located on the inner periphery surface of the outer cover portion 11.

Referring again to FIGS. 4 to 6, in the present embodiment, the upper portion of the main housing 30 may be formed as a main body portion 34. A first concentration space S1 into which an initial solution is introduced from the outside through an inlet member 20 may be provided inside the main body portion 34.

In the first concentration space S1, the intermediate solution may be generated by separating at least a portion of the first material from the initial solution. In this case, the first concentration space S1 may be formed to be suitable for a separation method for separating the first material. In the present embodiment, the first concentration space S1 may be extended in one direction, for example, in the up-down direction with reference to FIG. 6, to be suitable for a centrifugal separation method.

Meanwhile, the main body portion 34 may include a first main body part 35, an enlarged part 36, and a second main body part 37. The first main body part 35 and the second main body part 37 may be composed of tubular members that communicate with each other and are arranged in parallel.

In the illustrated embodiment, the first main body part 35 is formed of a cylindrical member positioned relatively lower, and the second main body part 37 is formed of a cylindrical member positioned relatively lower but having a larger diameter and longer length.

The enlarged part 36 connecting the two components may be provided between the first main body part 35 and the second main body part 37. In the present embodiment, the enlarged part 36 has an upwardly facing funnel shape so as to connect the first main body part 35 and the second main body part 37 having different diameters.

Hereinafter, a part of the first concentration space S1 provided inside the first main body part 35 is referred to as the first small space S1a, and the remaining part of the first concentration space S1 provided inside the enlarged part 36 and the second main body part 37 is referred to as the second small space S1b.

Meanwhile, the inner peripheral surface of the second main body part 37 may be provided with a second main housing-side screw thread 37a that may be coupled correspondingly to a first intermediate housing-side screw thread 41b of the intermediate housing 40 described later. In the illustrated embodiment, the second main housing-side screw thread 37a is located at the upper portion of the second main body part 37.

In this case, as will be described in detail later, the second main housing-side screw thread 37a may be formed over a considerable area along the length direction of the second main body part 37 so that the intermediate housing 40 may be screw-coupled and moved.

Meanwhile, the shape of the main body portion 34 is not limited to the illustrated embodiment, and the shape of the main body portion 34 may be variously modified as needed. For example, the diameters of the first small space S1a and the second small space S1b may be configured to be the same, or conversely, the first small space S1a may be configured to be larger than the second small space S1b.

Referring to FIGS. 4 to 6, the first concentration space S1 may be provided with the opening/closing member 50 that can open/close the first small space S1a and the second small space S1b. In the illustrated embodiment, the opening/closing member 50 is formed of a rod-shaped member that extends along the extension direction of the first concentration space S1, that is, in the up-down direction, and a stopper 52 having a predetermined thickness is provided at one end portion of the opening/closing member.

In the present embodiment, as the opening/closing member 50 moves up and down, the stopper 52 blocks or opens the portion where the first small space S1a and the second small space S1b are connected, so that the first small space S1a and the second small space S1b may be opened and closed. In this case, the intermediate housing 40 may be configured to support the opening/closing member 50 while being movably coupled to the main housing 30, so that the opening/closing member 50 may be moved up and down.

Referring to FIGS. 6 to 8, in the present embodiment, the intermediate housing 40 may be formed of a first intermediate body portion 41 and a second intermediate body portion 43 having a cylindrical shape. In this case, the first intermediate body portion 41 may have a diameter that is smaller than or equal to an inner diameter of the second main body part 37 so that the first intermediate body portion may be inserted into the second main body part 37 of the main housing 30, and the second intermediate body portion 43 may have a diameter that is larger than or equal to an outer diameter of the sub housing 70 so that the sub housing 70 described below may be inserted into the inside.

A step portion 42 connecting two components having different diameters may be provided between the first intermediate body portion 41 and the second intermediate body portion 43.

At least a part of the first intermediate body portion 41 may be inserted into the inside of the second main body part 37 of the main housing 30. In this case, the outer peripheral portion of the first intermediate body portion 41 may be movably coupled to the inner peripheral portion of the second main body part 37. To this end, the outer peripheral surface of the first intermediate body portion 41 may be provided with a first intermediate housing-side screw thread 41b coupled correspondingly to the second main housing-side screw thread 37a of the main housing 30 described above.

Accordingly, when the intermediate housing 40 is rotated in one direction by an external force, the first intermediate body portion 41 is inserted into the main housing 30, and the intermediate housing 40 may move toward the main housing 30. Conversely, when the intermediate housing 40 is rotated in another direction by an external force, the first intermediate body portion 41 is pulled out of the main housing 30, and the intermediate housing 40 may move away from the main housing 30.

In this case, the step portion 42 of the intermediate housing 40 may perform a function of limiting the relative movement distance of the main housing 30 and the intermediate housing 40. In the present embodiment, the step portion 42 interferes with the end portion of the second main body part 37 of the main housing 30 when the main housing 30 and the intermediate housing 40 come close by a certain distance. Accordingly, since the main housing 30 and the intermediate housing 40 may be prevented from coming too close, damage or failure of the solution concentration kit 1 may be minimized.

Meanwhile, a second sealing member groove 41a in which a second sealing member 46 may be installed may be provided on the outer peripheral surface of the first intermediate body portion 41. For example, the second sealing member 46 may be formed of an O-ring. In this case, a plurality of the second sealing members 46 and a plurality of the second sealing member grooves 41a may be provided along the extension direction of the first intermediate body portion 41. Of course, it may also be possible for the second sealing member grooves 41a to be formed on the inner peripheral surface of the second main body part 37.

On the inner peripheral surface of the second intermediate body portion 43, a second intermediate housing-side screw thread 43a that may be coupled correspondingly to the sub housing-side screw thread 72a of the sub housing 70 described later may be formed.

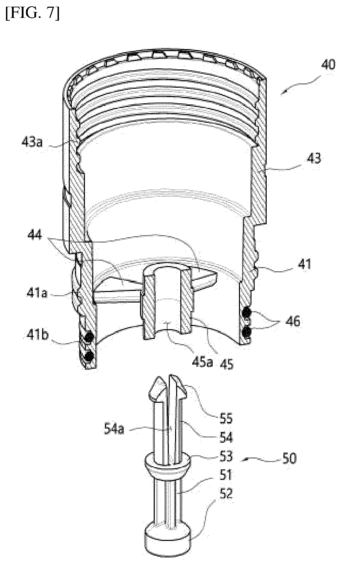

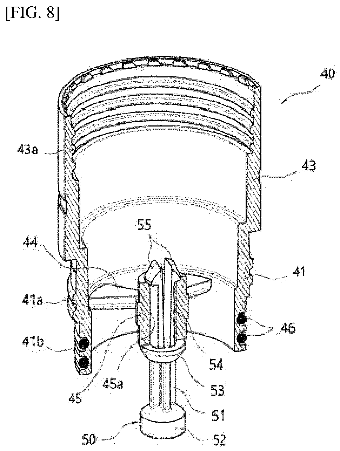

Meanwhile, referring to FIGS. 7 and 8, an opening/closing member coupling portion 45 may be provided inside the first intermediate body portion 41. An opening/closing member coupling hole 45a to which the opening/closing member 50 may be coupled may be formed in the up-down direction inside the opening/closing member coupling portion 45. In the illustrated embodiment, the opening/closing member coupling portion 45 is formed of a cylindrical member arranged in the up-down direction.

In this case, the opening/closing member support portion 44 may be provided between the opening/closing member coupling portion 45 and the inner peripheral surface of the first intermediate body portion 41. The opening/closing member support portion 44 may be formed of a bar or rib-shaped member for supporting the opening/closing member coupling portion 45 and the opening/closing member 50 coupled thereto. That is, one end portion of the opening/closing member support portion 44 may be connected to the first intermediate body portion 41 and the other end portion thereof may be connected to the opening/closing member coupling portion 45.

In this case, a plurality of the opening/closing member support portions 44 may be provided in a radial direction centered on the opening/closing member coupling portion 45. A predetermined fluid passing through the interior of the intermediate housing 40 may move from the lower part of the intermediate housing 40 to the upper part or from the upper part to the lower part through between the opening/closing member support portions 44.

Of course, the shape of the intermediate housing 40 is not limited to the shape described above, and the shape of the intermediate housing 40 may be appropriately modified depending on the shapes of the main housing 30 and sub housing 70 to be coupled and the method of movement of the opening/closing member 50 described later.

Referring to FIGS. 7 and 8, the opening/closing member 50 in the present embodiment may include an opening/closing member body portion 51, the stopper 52, and an elastic coupling portion 54. The opening/closing member body portion 51 is a rod-shaped member extending in one direction, and provides a base for other configurations of the opening/closing member 50.

As described above, the stopper 52 may be provided at one end portion of the opening/closing member body portion 51, for example, at the lower end portion with reference to FIG. 7. The stopper 52 may have a cylindrical shape with a diameter that is somewhat larger than that of the opening/closing member body portion 51, so as to facilitate opening and closing the first and second small spaces S1a and S1b described above.

The elastic coupling portion 54 may be extended outwardly, for example, upwardly, with reference to FIG. 7, from the other end portion of the opening/closing member body portion 51, for example, from the upper end portion with reference to FIG. 7. The elastic coupling portion 54 is configured to be fit-coupled to the opening/closing member coupling hole 45a of the opening/closing member coupling portion 45 described above.

In this case, the elastic coupling portion 54 may have a cut groove 54a formed in the extension direction so that the elastic coupling portion may be easily fit-coupled to the opening/closing member coupling hole 45a. In other words, the elastic coupling portion 54 may be formed of two bar parts having free ends.

When the elastic coupling portion 54 enters the opening/closing member coupling hole 45a, the two bar parts may be elastically deformed to be adjacent to each other and inserted into the opening/closing member coupling hole 45a. After the elastic coupling portion 54 is completely inserted into the opening/closing member coupling hole 45a, the restoring force of the two bar parts may strongly fix the opening/closing member 50 to the opening/closing member coupling portion 45.

In this case, in order to prevent the opening/closing member 50 from being detached from the opening/closing member coupling portion 45, an intermediate catch 53 protruding outwardly may be provided between the opening/closing member body portion 51 and the elastic coupling portion 54. The intermediate catch 53 is provided so as to be caught on one side of the opening/closing member coupling portion 45, thereby preventing the opening/closing member 50 from being pulled out to one side of the opening/closing member coupling portion 45. Similarly, an elastic catch 55 formed in a stepwise manner may be provided at an end portion of the elastic coupling portion 54 protruding outwardly from the opening/closing member coupling portion 45 so as to be caught on the other side portion of the opening/closing member coupling portion 45.

As described above, the opening/closing member 50 may be coupled with the intermediate housing 40 and move relative to the main housing 30 together with the intermediate housing 40.

Meanwhile, the opening/closing member 50 is not particularly limited in its shape or operation method as long as it can open/close the portion where the first small space S1a and the second small space S1b are connected so that the first small space S1a and the second small space S1b may be isolated (partitioned) or communicated. For example, in another embodiment, the opening/closing member 50 may be formed as a plate-shaped member that may move in a direction perpendicular to the extension direction of the first concentration space S1 and partition or communicate the first small space S1a and the second small space S1b.

Referring again to FIGS. 4 to 6, the sub housing 70 may be coupled to the second intermediate body portion 43 of the intermediate housing 40, and the isolation member 60 may be provided between the sub housing 70 and the intermediate housing 40.

As described above, the sub housing 70 is configured to separate at least a portion of the second material from the intermediate solution generated by separating at least a portion of the first material from the initial solution, thereby generating a concentrated solution in which the third material is concentrated.

The sub housing 70 may include a sub body portion 71 having a second concentration space S2 connected to the first concentration space S1 inside, an outlet portion 75 for forming a second flow path to connect the second concentration space S2 to the outside, and an outlet portion cover portion 76 surrounding the outside of the outlet portion 75. As will be described in detail later, an absorbent member 78 may be accommodated in the second concentration space S2.

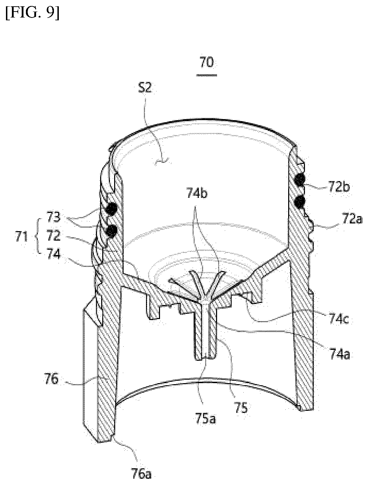

Referring to FIGS. 6 and 9 together, the sub body portion 71 may include a sub body part 72 and a capturing part 74. The sub body part 72 may be formed of a tubular member. In the illustrated embodiment, the sub body part 72 has a cylindrical shape extending in the up-down direction. In this case, a part of the second concentration space S2 is located inside the sub body part 72, and one side of the sub body part 72, the lower side with respect to FIG. 6, may be communicated with the second small space S1b of the first concentration space S1.

Meanwhile, as illustrated in FIG. 6, the sub body part 72 may be inserted into the inside of the second intermediate body portion 43 of the intermediate housing 40. Accordingly, the outer peripheral portion of the sub body part 72 and the inner peripheral portion of the second intermediate body portion 43 may come into contact with each other.

In this case, the sub housing-side screw thread 72a that may be coupled correspondingly to the second intermediate housing-side screw thread 43a of the above-described intermediate housing 40 may be formed on the outer peripheral surface of the sub body part 72. Accordingly, the intermediate housing 40 and the sub housing 70 may be coupled to each other.

On the outer peripheral surface of the sub body part 72, a third sealing member groove 72b in which a third sealing member 73 may be installed may be provided on one side of the sub housing-side screw thread 72a, for example, on the lower side with reference to FIG. 6. The third sealing member 73 may be formed of, for example, an O-ring. In this case, the third sealing member 73 and the third sealing member groove 72b may be provided in multiple along the extension direction of the sub body part 72. Meanwhile, the third sealing member groove 72b may also be configured to be formed on the inner peripheral surface of the second intermediate body portion 43.

The outlet portion 75 may be provided on one side of the sub body part 72, for example, on the upper side with reference to FIG. 6. The outlet portion 75 may be formed of a tubular member for forming a second flow path connecting the second concentration space S2 to the outside. In other words, the outlet portion 75 may be provided with an outlet hole 75a forming the second flow path.

Referring to FIG. 9, in the present embodiment, a capturing part 74 may be provided between the outlet portion 75 and the sub body part 72 to enable fluid within the second concentration space S2 to be captured. The capturing part 74 may have a shape in which the cross-sectional area decreases in one direction so that the fluid may be easily collected. In other words, the capturing part 74 may be formed as an inclined surface that slopes inwardly as it goes in one direction.

In this case, the one direction may be a direction toward the outlet portion 75 so that the collected fluid may be discharged to the outside. In the illustrated embodiment, the capturing part 74 has a funnel shape with an end portion positioned toward the outlet portion 75. Accordingly, one side of the second concentration space S2 adjacent to the capturing part 74 may also be formed into a corresponding funnel shape.

In this case, the capturing part 74 may be provided with an outlet 74a connected to the above-described outlet hole 75a. In the illustrated embodiment, the outlet 74a is formed in the center of the capturing part 74.

Meanwhile, on the inner surface of the capturing part 74 facing the second concentration space S2, a discharge groove 74b may be formed that extends in the direction of the flow of the fluid accommodated in the second concentration space S2 toward the outlet portion 75.

The discharge groove 74b may have a slit shape connected to the outlet 74a. In other words, the discharge groove 74b may have a shape that extends in the direction of the flow of the fluid flowing toward the outlet portion 75, that is, a shape in which the side portion in the extension direction is opened to the second concentration space S2.

Accordingly, even when the outlet 74a is covered by the absorbent member 78 accommodated in the second concentration space S2, the fluid in the second concentration space S2 may flow out to the outlet portion 75 through the discharge groove 74b.

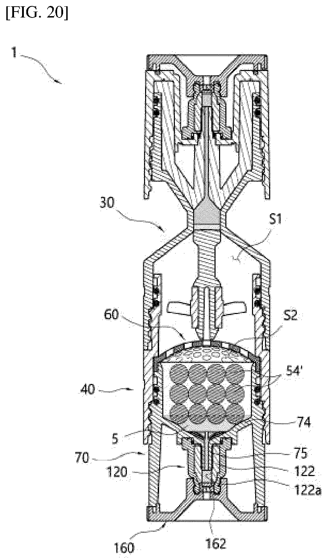

To this end, it is preferable that the width of the discharge groove 74b be formed to be smaller than the diameter of the absorbent member 78. Alternatively, it is preferable that the width of the discharge groove 74b be formed to be smaller than the diameter of the absorbent member 78' (as illustrated in FIG. 20) that has expanded by absorbing the second material.

Meanwhile, referring again to FIG. 6 and FIG. 9, a holder insertion groove 74c may be provided on the outer surface of the capturing part 74 to be coupled with the second coupling unit 120 described later. In this case, the holder insertion groove 74c may be formed along the circumferential direction centered on the outlet portion 75.

The outlet portion cover portion 76 may be provided outside the outlet portion 75. The outlet portion cover portion 76 is configured to protect the outlet portion 75 from being exposed to the outside and at the same time provide a base to which a part of the second sealing cap 160 described later may be coupled or supported.

The outlet portion cover portion 76 may extend outward from one side of the sub body part 72, the upper side with reference to FIG. 6. A sealing cap engaging groove 76a may be formed at the end portion in the extension direction of the outlet portion cover portion 76, on which a second sealing cap-side engaging protrusion 161a of the second sealing cap 160 may be engaged and supported.

Meanwhile, referring again to FIGS. 4 to 6, the isolation member 60 that partitions the second small space S1b of the first concentration space S1 and the second concentration space S2 may be provided between the intermediate housing 40 and the sub housing 70.

The isolation member 60 is configured to prevent the absorbent member 78 accommodated in the second concentration space S2 from moving from the second concentration space S2 to the first concentration space S1. Accordingly, the absorbent member 78 may be prevented from unintentionally leaving the second concentration space S2 and entering the first concentration space S1.

The isolation member 60 may include a plate-shaped partition portion 61 disposed between the first concentration space S1 and the second concentration space S2 to partition the first concentration space S1 and the second concentration space S2, and a coupling portion 62 provided along the periphery of the partition portion 61 so as to be coupled to the intermediate housing 40 and the sub housing 70.

The partition portion 61 may have a convex shape in the direction from the second concentration space S2 toward the first concentration space S1. In the illustrated embodiment, the partition portion 61 has a dome shape.

In this case, a flow hole 61a may be formed in the partition portion 61. The flow hole 61a may prevent the absorbent member 78 from moving from the second concentration space S2 to the first concentration space S1, while allowing the fluid, for example, the intermediate solution, in the first concentration space S1 to flow into the second concentration space S2.

To this end, it is preferable that the diameter of the flow hole 61a be formed to be smaller than the diameter of the absorbent member 78. Meanwhile, a plurality of the flow holes 61a may be provided throughout the entire area of the partition portion 61.

The coupling portion 62 provided around the periphery of the partition portion 61 may be configured to be fixedly coupled by being interposed between the second intermediate body portion 43 of the intermediate housing 40 and the sub body part 72 of the sub housing 70. In this case, in order to prevent the isolation member 60 from being separated toward the first concentration space S1 side, one side of the coupling portion 62 may be protruded outward so as to be caught by the step portion 42 of the intermediate housing 40.

Meanwhile, referring again to FIG. 6, the absorbent member 78 accommodated in the second concentration space S2 is configured to selectively absorb the second material as described above to generate a concentrated solution from the intermediate solution, and may be formed of a plurality of spherical structures.

In this case, the absorbent member 78 may be made of a super absorbent polymer (SAP) that may selectively absorb water as the second material. The super absorbent polymer refers to a material made of a hydrogel that can absorb water hundreds of times its dry weight.

The super absorbent polymer is made of porous materials with microscopic holes. The diameter of the microscopic holes formed in the super absorbent polymer is several nanometers, so it may selectively absorb only water molecules without absorbing large exosomes. In addition, the super absorbent polymer may also perform the function of selectively absorbing and removing impurities smaller than exosomes.

In order to compare the performance of the super absorbent polymer and an ultrafiltration device, the inventors of the present disclosure performed an experiment to produce exosome concentrate from 300 µl of plasma using the super absorbent polymer and ultrafiltration. The results are illustrated in [Table 1] below.

**[Table 1]**

| | Super absorbent polymer | Ultrafiltration |
|---|---|---|
| Cost ( 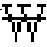 /rxn) | 28 | 6,908 |
| Device | - | Centrifugal separator (15,000 × g) |
| Time required (min) | 60 | 41 |
| Yield (%) | 74 | 62 |

Referring to Table 1, it can be confirmed that the unit cost of exosome production per unit rxn using super absorbent polymer is approximately 230 times cheaper than using ultrafiltration. These results suggest that exosome concentrate may be produced at a low cost by using the super absorbent polymer.

In addition, when using the super absorbent polymer, no separate device or apparatus or additional process is required, but when using ultrafiltration, a centrifuge and centrifugal separation process are required. These results suggest that by using the super absorbent polymer, the exosome concentrate may be easily produced without separate equipment.

In addition, it can be confirmed that the yield of the exosome concentrate is about 20% higher using the super absorbent polymer than using the ultrafiltration. These results suggest that the exosome concentrate may be produced with high efficiency by using the super absorbent polymer.

Furthermore, the inventors of the present disclosure conducted an experiment to compare the characteristics of the exosomes in the concentrate produced using the super absorbent polymer with those of the exosomes present in plasma before concentration, thereby confirming that producing the exosome concentrate using the super absorbent polymer does not affect the characteristics of the exosomes themselves.

In this way, the solution concentration kit 1 according to the embodiment of the present disclosure may simply produce the exosome concentrate from platelet rich plasma with high efficiency and low cost without separate equipment by using the super absorbent polymer.

Hereinafter, the coupling unit, sterilizing cap, and sealing cap according to the embodiment of the present disclosure will be described in more detail.

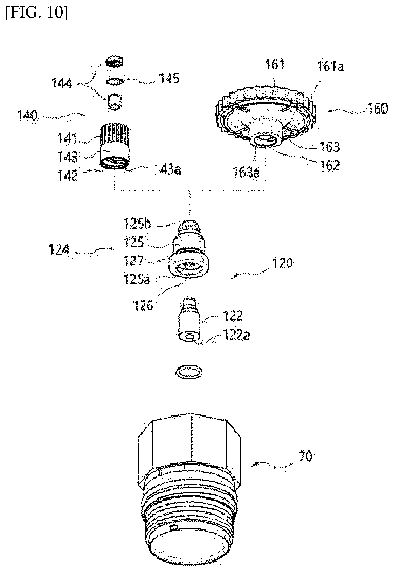

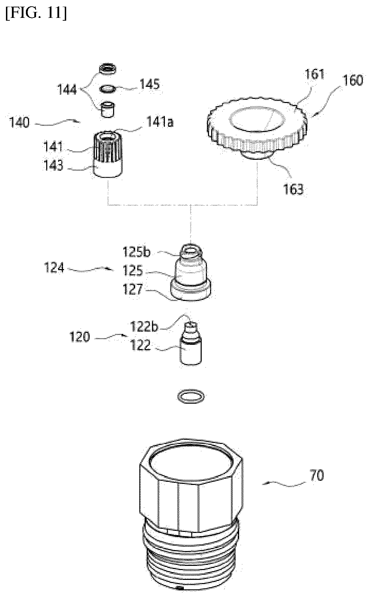

Referring to FIGS. 10 to 12, a second coupling unit 120 may be coupled to a sub housing 70 of a solution concentration kit according to an embodiment of the present disclosure. The second coupling unit 120 may perform a function of opening or closing the second flow path formed by the outlet portion 75 of the sub housing 70 and a function of connecting the sub housing 70 to the second sterilizing cap 140 and the second sealing cap 160 described below so as to be mutually connectable.

The second coupling unit 120 may include a second elastic body 122 and a second elastic body holder 124. The second elastic body 122 is configured to open or close the outlet portion 75 using elasticity, and for this purpose, may be made of a material such as rubber having a predetermined elasticity.

The second elastic body 122 may be provided with a second internal hole 122a with which the outlet portion 75 may be penetration-coupled. In the illustrated embodiment, the lower part of the second elastic body 122 has a cylindrical shape extending in one direction, and the second internal hole 122a described above is formed inside parallel to the extending direction. The upper part of the second elastic body 122 has a shape in which the cross-sectional area decreases toward the end portion, and a second slit line 122b cut in a straight or cross shape is provided on the upper surface of the second elastic body.

The second slit line 122b may be connected to the second internal hole 122a. Accordingly, when the second slit line 122b is opened by an external force, the outside and the second internal hole 122a may communicate with each other. When no external force is applied to the second elastic body 122, the second slit line 122b is closed by the elastic restoring force, so that the outside and the second internal hole 122a may be isolated from each other. In this way, according to the present embodiment, the first flow path formed by the outlet portion 75 of the sub housing 70 may be opened or closed by utilizing the elasticity of the second elastic body 122.

In addition, when an object such as a syringe is forcibly inserted into the second slit line 122b of the second elastic body 122, the inner surface of the second slit line 122b and the outer surface of the forcibly inserted object are in close contact with each other due to the elasticity of the second elastic body 122, so that external air may be prevented from entering the second internal hole 122a and the outlet portion 75 connected thereto, or fluid may be prevented from leaking to the outside from the outlet portion 75.

The second elastic body 122 may be coupled to the sub housing 70 by the second elastic body holder 124. The second elastic body holder 124 may include a second holder body portion 125 having a second elastic body installation hole 115a therein in which the second elastic body 122 may be installed.

The second holder body portion 125 may also perform the function of accommodating and protecting the second elastic body 122 inside. To this end, the second holder body portion 125 may be made of a material such as plastic having a predetermined rigidity.

The second elastic body installation hole 115a may have an inner peripheral portion corresponding to the outer shape of the second elastic body 122. In this case, the cross-section of the second elastic body installation hole 115a may be configured to have a size somewhat smaller than the cross-section of the second elastic body 122 so that the second elastic body 122 may be installed with a sense of fit.

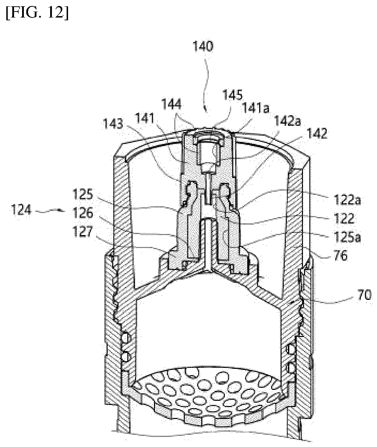

A second elastic body engaging protrusion 126 may be provided on one side of the second elastic body installation hole 115a, for example, on the lower side with reference to FIG. 12, to prevent the second elastic body 122 from falling downward. The second elastic body engaging protrusion 126 may have a shape that protrudes toward the center from the inner wall of a second elastic body installation hole 125a.

Meanwhile, referring to FIGS. 10 to 12, a second holder-side screw thread 125b that may be coupled correspondingly to any one of the second sterilizing cap-side screw thread 143a of the second sterilizing cap 140 and the second sealing cap-side screw thread 163a of the second sealing cap 160 described below may be formed on an outer peripheral surface of one side of the second holder body portion 125, for example, the upper side with reference to FIG. 10.

A second holder-side engaging protrusion 127 may be provided on the other side portion of the second holder body portion 125, for example, on the lower side with reference to FIG. 10. The second holder-side engaging protrusion 127 is configured to be inserted and coupled into the holder insertion groove 74c of the sub housing 70. The second holder-side engaging protrusion 127 may be formed along the lower perimeter of the second holder body portion 125 to correspond to the holder insertion groove 74c.

As described above, the second elastic body holder 124 may stably fix and connect the second elastic body 122 to the outlet portion 75 of the sub housing 70.

Meanwhile, referring again to FIGS. 10 to 12, the second sterilizing cap 140 may be coupled to the second coupling unit 120 coupled to the sub housing 70. The second sterilizing cap 140 is configured to forcibly inject external sterilizing gas into the sub housing 70 and the interior of the solution concentration kit by forming a second flow path communicating with the interior of the sub housing 70.

In the present embodiment, the second sterilizing cap 140 may be formed of a second sterilizing cap body portion 141 having a cylindrical shape. One surface of the second sterilizing cap body portion 141, in the illustrated embodiment, may be provided with a second filter coupling groove 141a into which a second filter member 144 and a second filter holder 145 may be inserted and coupled. The second filter coupling groove 141a may be formed to be recessed to correspond to the shape of the second filter holder 145 so that the second filter holder 145 may be inserted and arranged on the inside.

The second filter member 144 is configured to filter out foreign materials contained in the sterilizing gas entering the solution concentration kit through the second flow path, and the second filter holder 145 is configured to fixedly couple the second filter member 144 to the second filter coupling groove 141a. The second filter member 144 and the second filter holder 145 may be configured in various known configurations.

Meanwhile, the other surface of the second sterilizing cap body portion 141, for example, the lower surface with reference to FIG. 12, may be provided with a second injection tube 142 that protrudes downward. An injection hole 142a may be provided inside the second injection tube 142.

In this case, one opening of the second injection hole 142a may be located on the inner surface of the second filter coupling groove 141a so that the sterilizing gas filtered by the second filter member 144 may flow into the second injection hole 142a. It is preferable that the other opening of the second injection hole 142a be located at the end portion of the second injection tube 142.

The second injection tube 142 may be forcibly fit-coupled into the second slit line 122b of the second elastic body 122. Accordingly, the end portion of the second injection tube 142 is positioned in the second internal hole 122a of the second elastic body 122, so that the sterilizing gas filtered by the second filter member 144 may be forcibly injected into the outlet portion 75 and the inside of the solution concentration kit.

Meanwhile, a second sterilizing cap-side coupling portion 143 that may be coupled with a second elastic body holder 124 may be provided on the lower side of the second sterilizing cap body portion 141. The second sterilizing cap-side coupling portion 143 may extend downward to surround the outer side of the second injection tube 142. In the illustrated embodiment, the second sterilizing cap-side coupling portion 143 has a cylindrical shape that extends vertically.

The second sterilizing cap-side coupling portion 143 may be connected to the second elastic body holder 124 by wrapping around the outer surface of one side of the second elastic body holder 124. To this end, a second sterilizing cap-side screw thread 143a that may be coupled correspondingly to the second holder-side screw thread 125b described above may be formed on the inner peripheral surface of the second sterilizing cap-side coupling portion 143.

As described above, the second sterilizing cap 140 is coupled with the second coupling unit 120 to forcibly open the second slit line 122b of the second elastic body 122 to form a second flow path, thereby forcibly injecting the external sterilizing gas into the outlet portion 75 and the interior of the solution concentration kit. In this case, the second sterilizing cap 140 may inject the sterilizing gas from which foreign materials have been filtered into the interior of the solution concentration kit by using the second filter member 144.

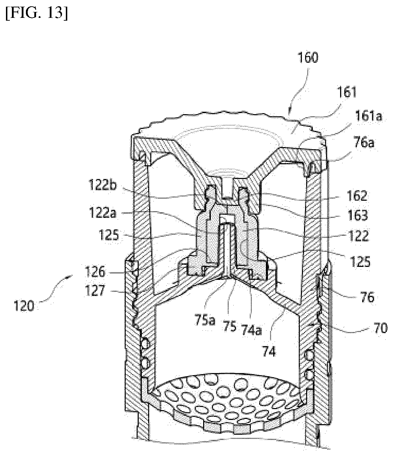

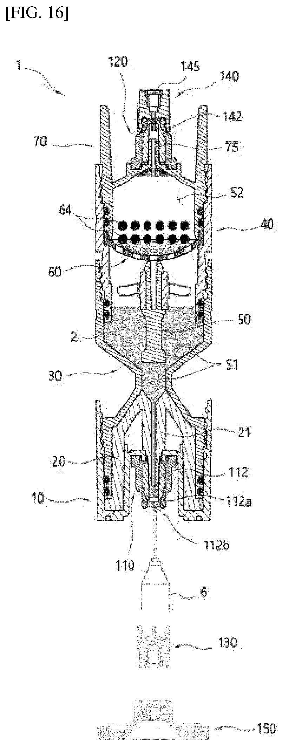

Meanwhile, referring to FIGS. 10, 11, and 13, the second sealing cap 160 may be coupled to the second coupling unit 120 coupled to the sub housing 70. The second sealing cap 160 is configured to isolate the interior of the sub housing 70 and the interior of the solution separation kit from the exterior by closing the second flow path that may communicate the interior of the sub housing 70 with the exterior. The solution separation kit, which is isolated from the exterior by the second sealing cap 160, may be ready to perform a predetermined process for producing the concentrated solution. This will be described later with reference to FIGS. 16 to 21.

The second sealing cap 160 may be formed of a second sealing cap body portion 161 having a disc shape so as to cover one side of the sub housing 70 entirely. However, the shape of the second sealing cap body portion 161 may be changed in various ways as needed.

On the surface of the second sealing cap body portion 161 facing the sub housing 70, for example, the lower surface with reference to FIG. 10, a second blocking portion 162 that protrudes convexly toward the sub housing 70 may be provided. The second blocking portion 162 is configured to block the second slit line 122b of the second elastic body 122.

In this case, it is preferable that the end portion of the second blocking portion 162 has a blunt shape so that the end portion may press one side of the second elastic body 122 on which the second slit line 122b is formed through a wide area. In the present embodiment, the second blocking portion 162 is configured so that the end portion includes a flat plane.

Accordingly, when the second sealing cap 160 is coupled to the second coupling unit 120 and the second blocking portion 162 covers one side of the second elastic body 122, the second slit line 122b is blocked, and the second internal hole 122a and the outlet hole 75a of the outlet portion 75 may be closed. In this case, when the second sealing cap 160 is coupled to the second coupling unit 120 and the second blocking portion 162 presses one side of the second elastic body 122, the inner surfaces of the second slit line 122b are in close contact with each other, and the second internal hole 122a and the outlet hole 75a of the outlet portion 75 may be closed more strongly.

Meanwhile, in the illustrated embodiment, a second sealing cap-side coupling portion 163 protruding downward along the circumference may be provided on the outer side of the second blocking portion 162 on the lower surface of the second sealing cap body portion 161.

The second sealing cap-side coupling portion 163 may be configured to cover one side of the second holder body portion 125 and couple the second holder body portion 125 and the second sealing cap 160. To this end, the second sealing cap-side screw thread 163a that may be coupled correspondingly to the second holder-side screw thread 125b described above may be formed on the inner side of the second sealing cap-side coupling portion 163.

Meanwhile, the second sealing cap-side engaging protrusion 161a may be formed along the circumferential direction (that is, the periphery direction) on the outer edge of the lower surface of the second sealing cap body portion 161. The second sealing cap-side engaging protrusion 161a is formed to protrude slightly in the downward direction, so that the second sealing cap-side engaging protrusion 161a may be fitted into the sealing cap engaging groove 76a of the sub housing 70.

As described above, the second sealing cap 160 is coupled with the second coupling unit 120 to block the second internal hole 122a of the second elastic body 122 and the outlet hole 75a of the outlet portion 75, thereby closing the second flow path and isolating the interior of the solution concentration kit from the exterior.

Meanwhile, referring again to FIGS. 4 to 6, the first coupling unit 110 may be coupled to the holder insertion groove 14b formed on the inner bottom portion 14 of the cover member 10 and the inlet portion 21 of the inlet member 20, and the first sterilizing cap 130 and the first sealing cap 150 may be selectively coupled to the first coupling unit 110.

The first coupling unit 110 may perform not only the function of opening or closing the first flow path formed by the inlet member 20, but also the function of connecting the first sterilizing cap 130 and the first sealing cap 150 described below so that they can be coupled with the inlet member 20 and the cover member 10.

In this case, the first coupling unit 110, the first sterilizing cap 130, and the first sealing cap 150 may be configured in the same manner as the second coupling unit 120, the second sterilizing cap 140, and the second sealing cap 160 described above, and therefore, the descriptions of the first coupling unit 110, the first sterilizing cap 130, and the first sealing cap 150 will be replaced with the descriptions of the second coupling unit 120, the second sterilizing cap 140, and the second sealing cap 160.

Hereinafter, an example of a method of using a solution concentration kit according to an embodiment of the present disclosure will be described.

Referring to FIG. 14, a solution concentration kit 1 according to an embodiment of the present disclosure may be packaged by being built into a separate packaging case 200, as illustrated in FIG. 14. The solution concentration kit 1 built into the packaging case 200 may perform a sterilization process using sterilizing gas in a sterilization chamber CH having an internal atmosphere in a reduced pressure state lower than atmospheric pressure.

This packaging case 200 may be made of a resin molding sheet 210 and a paper sheet 220. The resin molding sheet 210 may be provided with a recessed placement groove in which the solution concentration kit 1 may be placed. The paper sheet 220 may cover one open side of the placement groove. In this case, the outer edge of the paper sheet 220 may be sealed and bonded to the outer edge of the resin molding sheet 210.

It is preferable that the paper sheet 220 be made of pulp material having high gas permeability. Accordingly, a sterilizing gas such as ethylene oxide gas supplied into the internal space of the depressurized sterilization chamber CH may be injected into the solution concentration kit 1 by permeating the paper sheet 220.

To explain the sterilization process more specifically, the packaging case 200 in which the solution concentration kit 1 is stored is placed in a sterilization chamber CH, the internal pressure of the sterilization chamber CH is reduced to a pressure value lower than the atmospheric pressure so as to satisfy a pre-set negative pressure condition, and this reduced pressure state may be maintained for a certain period of time.

When the sterilizing gas is supplied into the interior of the sterilization chamber CH where reduced pressure is maintained, the supplied sterilizing gas may penetrate into the interior of the packaging case 200 by passing through the paper sheet 220 made of a material with high gas permeability.

The sterilizing gas that has penetrated into the interior of the packaging case 200 may be filled into the interior space of the solution concentration kit 1 consisting of an inlet member, a main housing, an intermediate housing, and a sub housing through the first and second injection tubes of the first and second sterilizing caps.

Accordingly, according to the present embodiment, the internal space where the initial solution is filled may be easily and completely sterilized with sterilizing gas while the assembled solution concentration kit 1 is stored in a finished product state inside the packaging case 200.

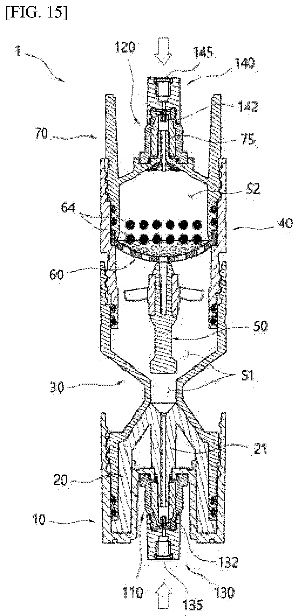

Referring to FIGS. 14 and 15 together, the sterilization process for sterilizing the solution concentration kit 1 according to the embodiment of the present disclosure described above may be performed in a state in which the first sterilizing cap 130 and the second sterilizing cap 140 are respectively coupled to the first coupling unit 110 and the second coupling unit 120, as illustrated in FIG. 15.

Since the first injection tube 132 of the first sterilizing cap 130 forms a first flow path with the inlet portion 21 of the inlet member 20, the first concentration space S1 may be communicated with the outside. Since the second injection tube 142 of the second sterilizing cap 140 forms the second flow path with the outlet portion 75 of the sub housing 70, the second concentration space S2 may be communicated with the outside.

The sterilizing gas supplied into the sterilization chamber CH in a reduced pressure atmosphere may be forcibly injected into the first and second concentration spaces S1 and S2 through the first and second flow paths, and this sterilizing gas may perform the sterilization process for sterilizing the interior of the solution concentration kit 1.

Meanwhile, this sterilization process may be performed in a state where the opening/closing member 50 is separated from the first small space S1a, and the first small space S1a and the second small space S1b communicate with each other.

Referring to FIG. 16, the process of introducing the initial solution into the main housing 30 may be performed in a state where the first sterilizing cap 130 is separated from the first coupling unit 110. Accordingly, the first slit line 112b of the first elastic body 112 may be exposed to the outside.

In this state, a predetermined object, for example, an injection syringe 6, may be forcibly inserted into the first slit line 112b of the first elastic body 112, thereby injecting an initial solution 2, for example, platelet rich plasma, into the first concentration space S1 inside the main housing 30.

The process of injecting the initial solution 2 into the main housing 30 may be performed in a state where the opening/closing member 50 is separated from the first small space S1a and the first small space S1a and the second small space S1b communicate with each other.

In this case, the outer peripheral portion of the needle of the injection syringe 6 is in close contact with the inner surface of the first slit line 112b due to the elasticity of the first elastic body 112, so that during the process of injecting the initial solution 2, external air may be prevented from coming into contact with the initial solution 2 or from being mixed into the first concentration space S1.

Meanwhile, a process of increasing the volume of the first concentration space S1 as needed and then injecting the initial solution 2 into the first concentration space S1 may be performed. For example, with reference to FIG. 16, by separating the cover member 10 and the inlet member 20 from the main housing 30 by a predetermined distance in the downward direction, the volume of the first concentration space S1 may be increased to a target amount, and then the initial solution 2 may be injected into the first concentration space S1 using an injection syringe 6.

When the initial solution 2 is completely injected into the first concentration space S1, the injection syringe 6 is removed, and the first sealing cap 150 is coupled to the first coupling unit 110, thereby closing the first flow path to prevent the initial solution 2 from leaking to the outside.

Thereafter, a process for separating the first material from the initial solution 2 may be performed in the first concentration space S1. For example, a centrifugal separation process for separating blood cells such as white blood cells and red blood cells from platelet rich plasma may be performed. Through this, at least a portion of the first material may be separated from the initial solution 2.

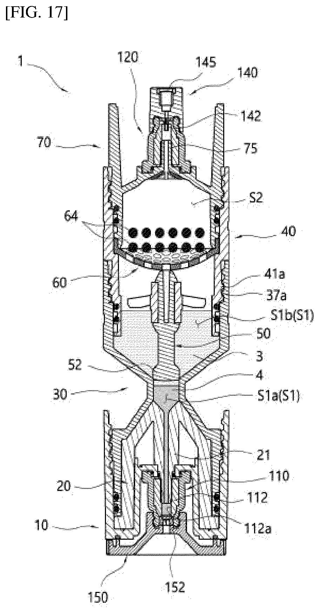

Referring to FIG. 17, when the centrifugal separation process is performed, an intermediate solution 3 in which at least a portion of the first material is separated from the initial solution and a first material layer 4 in which the first material is laminated may be separated by a difference in specific gravity. In the illustrated embodiment, the first material layer 4 is formed in the first small space S1a and most of the intermediate solution 3 is formed in the second small space S1b.

In this case, the intermediate solution 3 and the first material layer 4 separated by the centrifugal separation process may be distinguished with the naked eye by the colors exposed to the outside since the main housing 30, the intermediate housing 40, and the sub housing 70 are made of transparent materials.

Thereafter, as the intermediate housing 40 and the opening/closing member 50 move toward the first small space S1a, the portion where the first small space S1a and the second small space S1b are connected is closed, thereby isolating the first small space S1a and the second small space S1b. Accordingly, the first material layer 4 located in the first small space S1a may be prevented from mixing again with the intermediate solution 3 located in the second small space S1b.

Meanwhile, since the absorbent member 78 is prevented from moving from the second concentration space S2 to the first concentration space S1 by the isolation member 60, the absorbent member 78 is configured so as not to react first with the solutions located in the first concentration space S1 during the process described above.

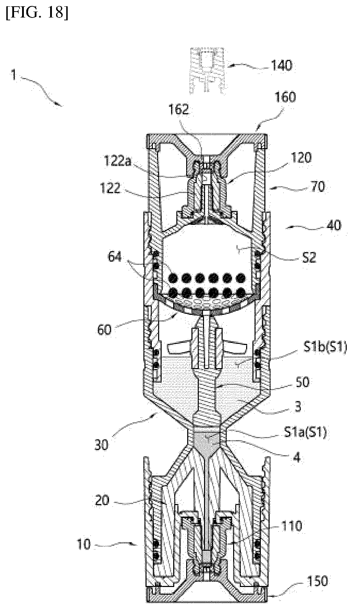

Referring to FIG. 18, by separating the second sterilizing cap 140 from the second coupling unit 120 and then coupling the second sealing cap 160, the second flow path may be closed so that the second concentration space S2 does not communicate with the outside.

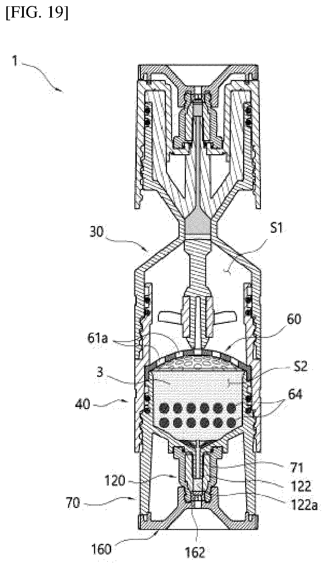

Referring to FIGS. 19 and 20 together, the arrangement of the solution concentration kit 1 may be appropriately adjusted so that the intermediate solution 3 may flow from the second small space S1b to the second concentration space S2. For example, the solution concentration kit 1 may be arranged in the up-down direction so that the intermediate solution 3 may be moved by gravity.

As the intermediate solution 3 and the absorbent member 78 meet and react in the second concentration space S2, the absorbent member 78 may selectively absorb the second material from the intermediate solution 3 to generate the concentrated solution 5 in which the third material is concentrated. For example, the second material may be water included in the platelet rich plasma, the absorbent member 78 may be made of a super absorbent polymer, and the third material to be concentrated may be exosomes. Meanwhile, the absorbent member 78' may selectively absorb the second material and expand.

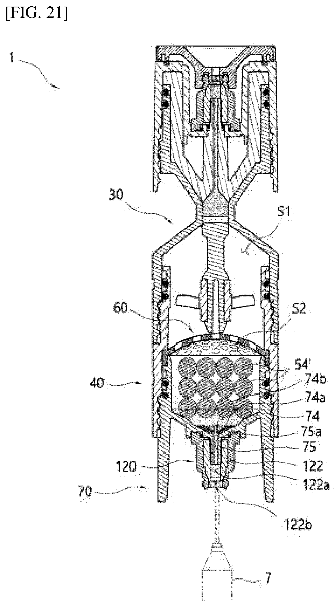

Referring to FIGS. 20 and 21 together, a process for extracting the concentrated solution 5 to the outside may be performed. This process may be performed by separating the second sealing cap 160 from the second coupling unit 120 so that the second slit line 122b of the second elastic body 122 is exposed to the outside.

In this state, a predetermined object, for example, an extraction syringe 7, may be forcibly inserted into the second slit line 122b of the second elastic body 122, thereby extracting a concentrated solution 5, for example, an exosome concentrate, from the second concentration space S2 inside the sub housing 70.

In this case, even when the outlet 74a of the capturing part 74 is covered by the expanded absorbent member 78', the concentrated solution 5 may be extracted to the outside through the discharge groove 74b connected to the outlet 74a.

Meanwhile, since the outer peripheral portion of the needle of the extraction syringe 7 is in close contact with the inner surface of the second slit line 122b due to the elasticity of the second elastic body 122, external air may be prevented from coming into contact with the concentrated solution 5 or from being mixed into the second concentration space S2 during the process of extracting the concentrated solution 5.

As previously discussed, according to the solution concentration kit according to the embodiment of the present disclosure, when the initial solution is injected into the main housing through the inlet member, the first material is separated inside the main housing and the second material is separated inside the sub housing, so that the concentrated solution in which the concentration of the third material increases may be easily and conveniently produced.

In this case, according to the solution concentration kit according to the embodiment of the present disclosure, the second material may be selectively separated using the absorbent member provided inside the sub housing, so that the concentrated solution may be easily and conveniently produced at low cost and with high efficiency without separate equipment.

Although the embodiments of the present disclosure have been described above, the spirit of the present disclosure is not limited to the embodiments presented in this specification, and those skilled in the art who understand the spirit of the present disclosure will be able to easily propose other embodiments by adding, changing, deleting, or adding components within the scope of the same spirit, but this will also be considered to fall within the spirit of the present disclosure.

## Claims

1. A kit for concentrating solution for generating, from an initial solution including a first material, a second material, and a third material, a concentrated solution in which a concentration of the third material increases, the kit for concentrating solution comprising:
a main housing having a first concentration space therein for separating at least a portion of the first material from the initial solution;
a sub housing having a second concentration space therein connected to the first concentration space; and
an absorbent member accommodated in the second concentration space so as to selectively absorb the second material.

2. The kit for concentrating solution of claim 1, wherein the absorbent member further includes an isolation member provided between the first concentration space and the second concentration space so that the absorbent member is not movable from the second concentration space to the first concentration space.

3. The kit for concentrating solution of claim 2, wherein the isolation member includes:
a plate-shaped partition portion that partitions the first concentration space and the second concentration space; and
a coupling portion formed around the partition portion so as to be coupled to the sub housing.

4. The kit for concentrating solution of claim 3, wherein a flow hole having a diameter smaller than a diameter of the absorbent member is formed in the partition portion so that the absorbent member does not pass through.

5. The kit for concentrating solution of claim 3, wherein the partition portion has a dome shape convex toward a side of the first concentration space.

6. The kit for concentrating solution of claim 1, wherein the sub housing includes:
a sub body portion having the second concentration space inside;
and
an outlet portion provided on one side of the sub body portion so that fluid flows out from the second concentration space to an outside.

7. The kit for concentrating solution of claim 6, wherein the sub body portion includes:
a tubular sub body part having one side connected to the first concentration space; and
a capturing part provided between the sub body part and the outlet portion so as to capture the fluid within the second concentration space, and
the capturing part is provided with an outlet connected to the outlet portion.

8. The kit for concentrating solution of claim 7, wherein the capturing part has a shape having a cross-sectional area that decreases toward a side of the outlet portion.

9. The kit for concentrating solution of claim 7, wherein a slit-shaped discharge groove formed in a flow direction of the fluid and connected to the outlet is formed in the capturing part.

10. The kit for concentrating solution of claim 9, wherein a width of the discharge groove is formed to be smaller than a diameter of the absorbent member.

11. The kit for concentrating solution of claim 10, wherein the width of the discharge groove is formed to be smaller than the diameter of the absorbent member expanded by absorbing the second material.

12. The kit for concentrating solution of claim 9, wherein a plurality of the discharge grooves is provided, and the plurality of discharge grooves is disposed radially from a center of the outlet.

13. The kit for concentrating solution of claim 12, further comprising:
an opening/closing member provided in the first concentration space; and
an intermediate housing provided between the main housing and the sub housing so as to move the opening/closing member,
wherein the first concentration space is partitioned into a first small space and a second small space disposed closer to the second concentration space than the first small space, and
the opening/closing member is provided so as to open/close a portion where the first small space and the second small space are connected.

14. The kit for concentrating solution of claim 13, wherein the intermediate housing includes:
a first intermediate body portion which is formed of a hollow member inserted into the main housing and includes an outer peripheral portion movably coupled to an inner wall of the main housing;
a second intermediate body portion provided on one side of the first intermediate body portion to be coupled to the sub housing; and
an opening/closing member support portion provided inside the intermediate housing to support the opening/closing member.

15. The kit for concentrating solution of claim 14, wherein corresponding screw threads are formed on the outer peripheral portion of the first intermediate body portion and the inner wall of the main housing so as to be screw-coupled.

16. The kit for concentrating solution of claim 1, wherein an inlet pipe forming a first flow path for fluidly communicating between the first concentration space and the outside is provided on one side of the main housing,
an outlet pipe forming a second flow path for fluidly communicating between the second concentration space and the outside is provided on one side of the sub housing,
a first elastic body having a first slit line formed for opening and closing the first flow path is coupled to an end portion of the inlet pipe, and
a second elastic body having a second slit line formed for opening and closing the second flow path is coupled to an end portion of the outlet pipe.

17. The kit for concentrating solution of claim 16, further comprising:
a first sterilizing cap including a first sterilizing cap body that is coupled to the one side of the main housing and a first injection tube that protrudes from the first sterilizing cap body and is penetration-coupled to the first slit line so that the first flow path is opened to the outside; and
a second sterilizing cap including a second sterilizing cap body that is coupled to the one side of the sub housing and a second injection tube that protrudes from the second sterilizing cap body and is penetration-coupled to the second slit line so that the second flow path is opened to the outside.

18. The kit for concentrating solution of claim 16, further comprising:
a first sealing cap including a first sealing cap body that is coupled to the one side of the main housing and a first blocking member that protrudes from the first sealing cap body and blocks the first slit line so that the first flow path is closed; and
a second sealing cap including a first sealing cap body that is coupled to the one side of the sub housing and a second blocking member that protrudes from the first sealing cap body and blocks the second slit line so that the second flow path is closed.

19. The kit for concentrating solution of claim 1, wherein the absorbent member includes a plurality of structures having a spherical shape.

20. The kit for concentrating solution of claim 1, wherein the second material is water, and
the absorbent member is made of a super absorbent polymer (SAP) that selectively absorbs water.

21. The kit for concentrating solution of claim 20, wherein the initial solution includes platelet rich plasma (PRP), and
the third material includes an exosome included in the platelet rich plasma.

22. The kit for concentrating solution of claim 21, wherein the first material includes blood cells remaining in the platelet rich plasma, and
the first concentration space is formed to extend in one direction to separate the first material from the initial solution by centrifugal separation method.
